# EUROPEAN PATENT SPECIFICATION

(11) **EP 1 012 177 B1**
(45) Date of publication and mention of the grant of the patent: **09.08.2006**
(21) Application number: 98945911.0
(22) Date of filing: 04.09.1998
(51) Int. Cl.: C12N 15/12, C07K 14/72, C12N 5/10, G01N 33/50

(54) **ESTROGEN RECEPTOR**
ÖSTROGENREZEPTOR
RECEPTEUR D'OESTROGENE

(30) Priority: 08.09.1997 US 58271 P; 30.09.1997 US 60520 P; 30.10.1997 GB 9722884; 20.03.1998 GB 9806032
(43) Date of publication of application: 28.06.2000
(73) Proprietor: Merck & Co., Inc., Rahway, New Jersey 07065 (US)
(72) Inventor: WILKINSON, Hilary, Rahway, NJ 07065 (US)
(74) Representative: Horgan, James Michael Frederic
(86) International application number: PCT/US1998/018577
(87) International publication number: WO 1999/012961

(56) References cited:
- EP-A- 0 798 378
- WO-A-97/09348
- WO-A-99/07847
- WO-A-99/11760
- DATABASE EMBL 14 November 1996 (1996-11-14), GAUGHAN, J.: "C. jacchus mRNA for estrogen receptor beta" XP002291601 Database accession no. Y09372
- KUIPER et al., "Cloning of a Novel Estrogen Receptor Expressed in Rat Prostate and Ovary", PROC. NATL. ACAD. SCI. USA, 11 June 1996, Vol. 93, No. 12, pages 5925-5930, XP002915519
- MOSSELMAN et al., "ERbeta: Identification and Characterization of a Novel Human Estrogen Receptor", FEBS LETTER, 19 August 1996, Vol. 392, No. 1, pages 49-53, XP002915520
- WILKINSON H.A. ET AL: 'Identification and Characterization of a Functionally Distinct Form of Human Estrogen Receptor beta' ENDOCRINOLOGY vol. 143, no. 4, 2002, pages 1558 - 1561
- XU L. ET AL: 'Human Estrogen Receptor beta 548 Is Not a Common Variant in Three Distinct Populations' ENDOCRINOLOGY vol. 144, no. 8, 2003, pages 3541 - 3546

## Description

### FIELD OF THE INVENTION

This invention relates to a novel estrogen receptor and to the polynucleotide sequences encoding this receptor. This invention also relates to methods for identifying ligands which bind to this receptor. The ligands so identified, pharmaceutical compositions comprising such ligands, and pharmaceutical compositions useful for treating or preventing estrogen receptor mediated diseases or conditions are also contemplated.

### BACKGROUND OF THE INVENTION

Nuclear receptors are a large class of proteins that are responsible for the regulation of complex cellular events including cell differentiation, homeostasis, the growth and functioning of various organs and tissues, and transcription. It is believed that nuclear receptors function by transducing extracellular chemical signals from hormones into a transcriptional response.

Estrogen receptors are a subclass of the larger nuclear receptor class. The estrogen receptors are proteins that are responsive to estrogen and estrogen-like molecules. Estrogen receptors are believed to play an important role in the mammalian endocrine system, the reproductive organs, breast tissue, bone tissue, and the vascular system, and are believed to be involved in the development and progression of various disease states such as abnormal bone resorption, cardiovascular disease, cancer, and central nervous system disorders. It is believed that various disease states and conditions can be treated or prevented by the development of appropriate ligands, i.e. drugs, for modifying the activity of estrogen receptors. Consequently there is a need to identify estrogen receptors and their mode of action and to also identify ligands for modifying the action of these receptors.

At least two distinct types of estrogen receptors have been reported. An estrogen receptor having 595 amino acids is disclosed in Green, S. et al., *Nature*, 320, pp. 134-139 (1986) and Greene, G.L. et al., *Science,* 231, pp. 1150-1154 (1986).

These references also disclose the corresponding DNA sequences for the receptor.

The other reported type of estrogen receptor has been disclosed by two research groups and has been designated "β" (beta). One research group discloses a 485 amino acid β receptor that is obtained from rat, human, and mouse sources, as well as the corresponding DNA sequences. *See* PCT application No. WO 97/09348, to Kuiper, G.G. J. M. et al., published March 13, 1997.

The second research group discloses a similar estrogen receptor containing 483 amino acids. The corresponding DNA sequence is also disclosed. *See* Mosselman, S. et al., *ERβ: identification and characterization of a novel human estrogen receptor, FEBS Letters,* 392, pp. 49-53 (1996).

In the present invention, a novel estrogen receptor having 548 amino acid units, and that is distinct from the disclosed 595 amino acid, 485 amino acid, and 483 amino acid estrogen receptors, has been identified and isolated from human tissue. It is believed that this novel estrogen receptor plays a key role in mammalian physiology. This novel estrogen receptor is an important research tool for identifying and designing ligands for use in pharmaceutical compositions for treating and/or preventing a wide range of estrogen receptor mediated diseases or conditions.

It is therefore an object of the present invention to provide a novel isolated estrogen receptor.

It is another object of the present invention to provide the amino acid sequence of a novel estrogen receptor.

It is another object of the present invention to provide the polynucleotide sequence encoding a novel estrogen receptor. It is another object of the present invention to provide methods for producing a novel estrogen receptor.

It is another object of the present invention to provide methods for identifying ligands capable of binding to a novel estrogen receptor.

There are also disclosed pharmaceutical compositions comprising ligands capable of binding to a novel estrogen receptor.

There are also disclosed methods for treating and/or preventing estrogen receptor mediated diseases or conditions.

These and other objects will become readily apparent from the detailed description which follows.

### SUMMARY OF THE INVENTION

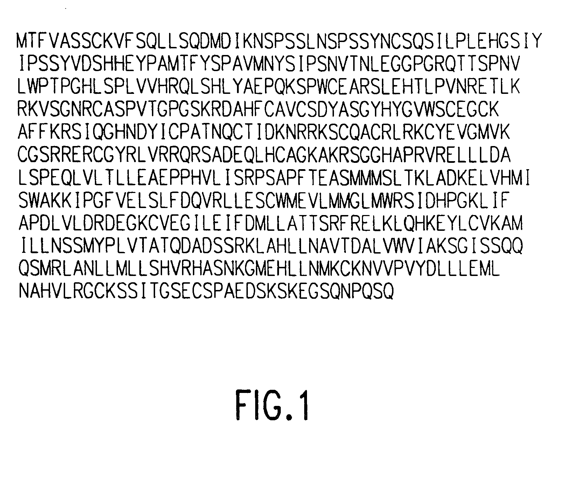

The present invention relates to an isolated estrogen receptor comprising the amino acid sequence of Figure 1 (which also corresponds to SEQ ID NO: 1).

There is also disclosed an isolated estrogen receptor having an amino acid sequence that is substantially similar to the amino acid sequence of Figure 1, wherein the estrogen receptor comprises at least 531 amino acids.

There is also disclosed an isolated estrogen receptor comprising at least 531 amino acids and having substantially the same ligand binding properties or substantially the same DNA binding properties as the estrogen receptor of Figure 1.

In further embodiments, the present invention relates to an isolated estrogen receptor that is derived from mammalian cells, preferably human cells.

In further embodiments, the present invention relates to an isolated polynucleotide encoding the estrogen receptor having the amino acid sequence of Figure 1.

In further embodiments, the present invention relates to an isolated polynucleotide which is a DNA or a cDNA.

There is also disclosed an isolated polynucleotide which hydridizes to and is complementary to the polynucleotide encoding the estrogen receptor having the amino acid sequence of Figure 1.

There is also disclosed an isolated polynucleotide comprising a polynucleotide encoding a mature polypeptide encoded by the estrogen receptor polynucleotide contained in an ATCC Deposit selected from the group consisting of ATCC Deposit No. 209238, ATCC Deposit No. 209239, and ATCC Deposit No. 209240.

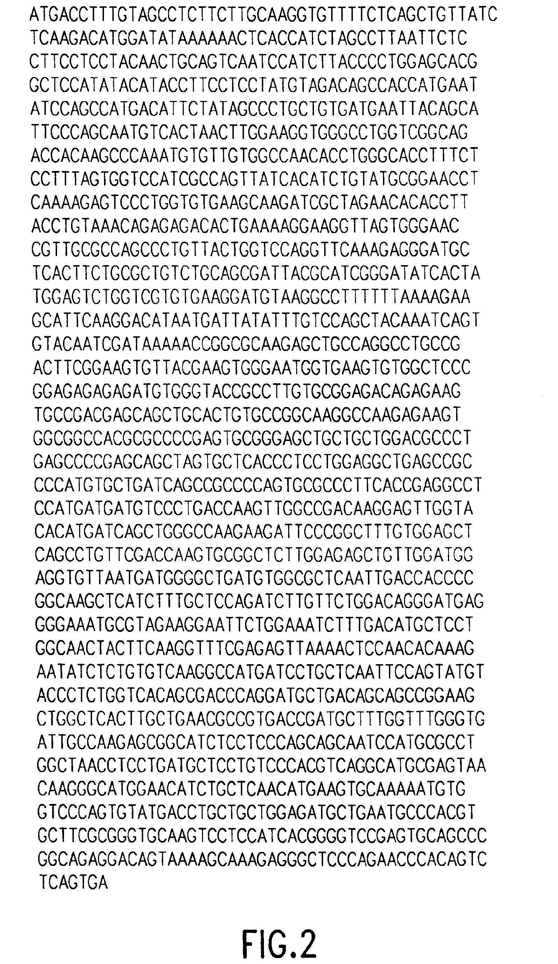

In further embodiments, the present invention relates to an isolated polynucleotide comprising the nucleotide sequence of Figure 2 (which also corresponds to SEQ ID NO: 2).

There is also disclosed an isolated polynucleotide which hybridizes to and is complementary to the polynucleotide of Figure 2, wherein said polynucleotide comprises at least 1593 nucleotides.

In further embodiments, the present invention relates to a vector containing the DNA.

In further embodiments, the present invention relates to a host cell transformed or transfected with the vector of the present invention.

In further embodiments, the present invention relates to a method for producing an estrogen receptor of the present invention.

In further embodiments, the present invention relates to a method for determining whether a ligand can bind to the estrogen receptor of the present invention.

There is also disclosed a ligand detected by the methods of the present invention.

There is also disclosed a pharmaceutical composition comprising a ligand of the present invention.

There is also disclosed a method for treating or preventing an estrogen receptor mediated disease or condition by administering an effective amount of a pharmaceutical composition as disclosed herein.

The deposits referred to herein will be maintained under the Budapest Treaty on the International Recognition of the Deposit of Microorganisms for the purposes of Patent Procedure.

The sequence of the polynucleotides contained in the deposited materials, as well as the amino acid sequence of the polypeptides encoded thereby, may be controlling in the event of any conflict with the description of the sequences herein.

All percentages and ratios used herein, unless otherwise indicated, are by weight. The invention hereof can comprise, consist of, or consist essentially of the essential as well as optional ingredients, components, and methods described herein.

### BRIEF DESCRIPTION OF THE FIGURES

FIG. 1 shows the amino acid sequence of the estrogen receptor, i.e. the polypeptide, of the present invention.
FIG. 2 shows the nucleotide sequence, i.e. the cDNA polynucleotide, encoding the estrogen receptor of the present invention. This sequence includes the translation termination codon "TGA".

### DESCRIPTION OF THE INVENTION

In accordance with one aspect of the present invention, there is provided a polypeptide, namely an estrogen receptor, which has the deduced amino acid sequence of FIG. 1. Fragments, analogs and derivatives of such an estrogen receptor are also contemplated

The terms "fragments", "derivatives", and "analogs" when referring to the estrogen receptor of FIG. 1 or that encoded by the deposited DNA, means a polypeptide which retains essentially the same biological function or activity as such estrogen receptor. Thus, an analog includes a proprotein which can be activated by cleavage of the proprotein portion to produce an active mature estrogen receptor.

The estrogen receptor of the present invention can be a recombinant polypeptide, a natural polypeptide, or a synthetic polypeptide of the sequence of FIG. 1.

Also contemplated are splice variants of the receptor of FIG. 1.

The fragments, derivatives, or analogs of the estrogen receptor of FIG. 1 or that encoded by the deposited DNA can be (i) one in which one or more of the amino acid residues are substituted with a conserved or non-conserved amino acid residue (preferably a conserved amino acid residue) and such substituted amino acid residue can be one that is or is not encoded by the genetic code, or (ii) one in which one or more of the amino acid residues includes a substituent group, or (iii) one in which the mature estrogen receptor is fused with another compound, such as a compound to increase the half-life of the estrogen receptor (for example, polyethylene glycol), or (iv) one in which the additional amino acids are fused to the mature estrogen receptor, such as a leader or secretory sequence or a sequence which is employed for purification of the mature estrogen receptor or a proprotein sequence. Such fragments, derivatives and analogs are deemed to be within the scope of those skilled in the art from the teachings herein.

There are also disclosed estrogen receptors which have substantially the same amino acid sequence as the estrogen receptor of Figure 1.

There are also disclosed estrogen receptors comprising at least 531 amino acids and having substantially the same ligand binding properties or substantially the same DNA binding properties as that of the estrogen receptor of Figure 1. In other words, the respective ligand binding or DNA binding domains of the receptors have at least about 75%, homology, preferably about 90% homology, more preferably about 95% homology, and most preferably about 99% homology to each of the respective ligand binding and DNA binding domains in the receptor of Figure 1.

In accordance with another aspect of the present invention, there is provided an isolated nucleic acid, i.e. the polynucleotide, which encodes for the mature estrogen receptor having the deduced amino acid sequence of FIG. 1.

A polynucleotide encoding an estrogen receptor of the present invention can be obtained by performing polymerase chain reactions (PCR) on human testis cDNA and subcloning into a vector in JM109 E. coli. Alternatively, the polynucleotide can be obtained by screening a human genomic DNA library derived from human testis.

The polynucleotide of the present invention can be in the form of RNA or in the form of DNA, which DNA includes cDNA, genomic DNA, and synthetic DNA. The DNA can be double-stranded or single-stranded, and if single stranded can be the coding strand or non-coding (anti-sense) strand. The coding sequence which encodes the mature estrogen receptor can be identical to the coding sequence shown in FIG. 2 or that of the deposited clones or can be a different coding sequence, which coding sequence, as a result of redundancy or degeneracy of the genetic code, encodes the same, mature estrogen receptors as the DNA of FIG. 2 or the deposited DNA.

The polynucleotide which encodes for the mature estrogen receptor of FIG. 1 or for the mature polypeptide encoded by the deposited DNA can include: only the coding sequence for the mature polypeptide; the coding sequence for the mature polypeptide and additional coding sequence such as a leader or secretory sequence or a proprotein sequence; or the coding sequence for the mature polypeptide (and optionally additional coding sequence) and non-coding sequence, such as introns or non-coding sequence 5' and/or 3' of the coding sequence for the mature polypeptide.

Thus, the term "polynucleotide encoding a polypeptide" encompasses a polynucleotide which includes coding sequence for the polypeptide as well as a polynucleotide which includes additional coding and/or non-coding sequence.

There are also disclosed variants of the hereinabove described polynucleotides which encode for fragments, analogs and derivatives of the polypeptide having the deduced amino acid sequence of FIG. 1 or the polypeptide encoded by the DNA of the deposited clones. The variant of the polynucleotide can be a naturally occurring allelic variant of the polynucleotide. There are also disclosed polynucleotide probes constructed from the polynucleotide sequence of FIG. 2 or a segment of the sequence of FIG. 2 amplified by the PCR method, which can be utilized to screen a cDNA library to deduce the estrogen receptor of the present invention.

Thus, the present invention includes polynucleotides encoding the same mature estrogen receptor as shown in FIG. 1. Variants of such polynucleotides which variants encode for fragments, derivatives or analogs of the polypeptide of FIG. 2 are also contemplated.

Such nucleotide variants include deletion variants, substitution variants and addition or insertion variants.

As hereinabove indicated, the polynucleotide can have a coding sequence which is a naturally occurring allelic variant of the coding sequence shown in FIG. 2 or of the coding sequence of the deposited clones. As known in the art, an allelic variant is an alternate form of a polynucleotide sequence which can have a substitution, deletion or addition of one or more nucleotides, which does not substantially alter the function of the encoded polypeptide.

There are also disclosed polynucleotides which hybridize to the polynucleotides encoding the estrogen receptor having the amino acid sequence of FIG. 1. There is disclosed an isolated polynucleotide which hybridizes to and is at least about 75% complementary to the polynucleotide encoding the estrogen receptor having the amino acid sequence of FIG. 1. There is disclosed an isolated polynucleotide which hybridizes to and is at least about 90% complementary to the polynucleotide encoding the estrogen receptor having the amino acid sequence of FIG. 1. There is disclosed an isolated polynucleotide which hybridizes to and is at least about 95% complementary to the polynucleotide encoding the estrogen receptor having the amino acid sequence of FIG. 1. There is disclosed an isolated polynucleotide which hybridizes to and is at least about 99% complementary to the polynucleotide encoding the estrogen receptor having the amino acid sequence of FIG. 1.

There is disclosed an isolated polynucleotide comprising at least 1593 nucleotides. There is disclosed an isolated polynucleotide comprising at least 1593 nucleotides which hybridizes to and is at least about 75% complementary to the polynucleotide of FIG. 2. There is disclosed an isolated polynucleotide comprising at least 1593 nucleotides which hybridizes to and is at least about 90% complementary to the polynucleotide of FIG. 2. There is disclosed an isolated polynucleotide comprising at least 1593 nucleotides which hybridizes to and is at least about 95% complementary to the polynucleotide of FIG. 2. There is disclosed an isolated polynucleotide which hybridizes to and is at least about 99% complementary to the polynucleotide of FIG. 2.

The polynucleotides which hybridize to the hereinabove described polynucleotides encode estrogen receptors which retain substantially the same biological function or activity as the mature estrogen receptors encoded by the cDNA of FIG 2 or the deposited DNA. Hybridization is described in U.S. Patent No. 5,501,969, to Hastings et al., issued March 26, 1996.

The polypeptides and polynucleotides of the present invention are provided in an isolated form, and preferably are purified to homogeneity.

The term "isolated' means that the material is removed from its original environment (e.g., the natural environment if it is naturally-occuring). For example, a naturally-occuring polynucleotide or polypeptide present in a living animal is not isolated, but the same polynucleotide or DNA or polypeptide, separated from some or all of the coexisting materials in the natural system, is isolated. Such polynucleotide could be part of a vector and/or such polynucleotide or polypeptide could be part of a composition, and still be isolated in that such vector or composition is not part of its natural environment.

The present invention also relates to vectors which include polynucleotides of the present invention, host cells which are genetically engineered with vectors of the invention and the production of estrogen receptors of the invention by recombinant techniques.

Host cells are genetically engineered (transduced or transformed or transfected) with the vectors of this invention which can be, for example, a cloning vector or an expression vector. The vector can be, for example in the form of a plasmid, a viral particle, a phage, etc. The engineered host cells can be cultured in conventional nutrient media mddified for activating promoters, selecting transformants or amplifying the estrogen receptor genes. The culture conditions, such as temperature, pH and the like, are those previously used with the host cell selected for expression, and will be apparent to the ordinarily skilled artisan.

The polynucleotide of the present invention can be employed for producing a polypeptide by recombinant techniques. Thus, for example, the polynucleotide sequence can be included in any one of a variety of expression vehicles, in particular vectors or plasmids for expressing an estrogen receptor. Such vectors include chromosomal, nonchromosomal and synthetic DNA sequences, e.g., derivatives of SV40: bacterial plasmids; phage DNA; yeast plasmids; vectors derived from combinations of plasmids and phage DNA, viral DNA such as vaccinia, adenovirus, fowl, pox virus, and pseudorabies. However, any other plasmid or vector can be used as long as it is replicable and viable in the host.

As hereinabove indicated the appropriate DNA sequence can be inserted into the vector by a variety of procedures. In general, the DNA sequence is inserted into appropriate restriction endonuclease sites by procedures known in the art. Such procedures and others are deemed to be within the scope of those skilled in the art.

The present invention also includes recombinant constructs comprising one or more of the sequences of the present invention. The constructs comprise a vector, such as a plasmid or viral vector, into which a sequence of the invention has been inserted, in a forward or reverse orientation. Large numbers of suitable vectors and promoters are known to those of skill in the art, and are commercially available.

In a further embodiment, the present invention relates to host cells containing the above-described construct. The host cell can be a higher eukaryotic cell, such as a mammalian cell, or a lower eukaryotic cell, such as a yeast cell, or the host cell can be a prokaryotic cell, such as a bacterial cell. Introduction of the construct into the host cell can be effected by calcium phosphate transfection, DEAE-Dextran mediated transfection, or electroporation (Davis, L., Dibner, M., Battey, I., *Basic Methods in Molecular Biology,* 1986, which is incorporated by reference herein in its entirety).

The constructs in host cells can be used in a conventional manner to produce the gene product encoded by the recombinant sequence. Alternatively, the estrogen receptors of the present invention can be synthetically produced by conventional peptide synthesizers.

Mature estrogen receptors can be expressed in mammalian cells, yeast, bacteria, or other cells under the control of appropriate promoters. Cell-free translation systems can also be employed to produce such estrogen receptors using RNAs derived from the DNA constructs of the present invention. Appropriate cloning and expression vectors for use with prokaryotic and eukaryotic hosts are described by Sambrook et al., *Molecular Cloning: A Laboratory Manual,* Second Edition (Cold Spring Harbor, NY, 1989).

The estrogen receptors of the present invention can be naturally purified products expressed from a high expressing cell line, or a product of chemical synthetic procedures, or produced by recombinant techniques from a prokaryotic or eukaryotic host (for example, by bacterial, yeast, higher plant, insect and mammalian cells in culture). Alternatively, a baculovirus/insect cell expression system can also be employed.

The estrogen receptors, their fragments or other derivatives or analogs thereof, or cells expressing them can be used as an immunogen to produce antibodies thereto. These antibodies can be, for example, polyclonal or monoclonal antibodies. There are also disclosed chimeric, single chain and humanized antibodies, as well as Fab fragments, or the product of a Fab expression library- Various procedures known in the art can be used for the production of such antibodies and fragments.

There are also disclosed ligands, i.e. drugs, of the estrogen receptors herein. The term "ligand" as used herein means any molecule which binds to the estrogen receptor of the present invention. These ligands can have either agonist, partial agonist, antagonist, partial antagonist, inverse agonist; or mixtures of these properties. Thus, for example, a ligand that binds to an estrogen receptor of the present invention might modify, inhibit, or eliminate its function. In this way, the ligand can be used to treat or prevent a disease in which the estrogen receptor is involved. The ligands contemplated herein are those that have selectivity to specifically activate or inhibit genes that are normally regulated by the estrogen receptors of the present invention.

The present invention also relates to methods for determining whether a ligand not known to be capable of binding to a human estrogen receptor can bind to a human estrogen receptor. These methods comprise contacting a mammalian cell comprising an isolated DNA molecule encoding a human estrogen receptor with the ligand under conditions permitting binding of ligands known to bind to an estrogen receptor, detecting the presence of any of the ligand bound to a human estrogen receptor, and thereby determining whether the ligand binds to a human estrogen receptor. In these methods, the mammalian cell is actually expressing the isolated DNA molecules. The general methodology for conducting such a method is well known to those of ordinary skill in the art. *See* EP 787,797, to Weinshank et al., published July 6, 1997. Alternatively, RNA that ultimately encodes for the estrogen receptor could be injected into, for example Xenopus oocytes, and expressed, and used in analogous assay experiments.

There are also disclosed pharmaceutical compositions comprising the ligands. Such compositions comprise a pharmaceutically effective amount of the ligand. The term "pharmaceutically effective amount", as used herein, means that amount of the ligand that will elicit the desired therapeutic effect or response when administered in accordance with the desired treatment regimen. The ligand is typically administered in admixture with suitable pharmaceutical diluents, excipients, or carriers, collectively referred to herein as "carrier materials", suitably selected with respect to the mode of administration, i.e. oral, I.V., nasal, parenteral, ocular, etc. A wide variety of product and dosage forms well known to one of ordinary skill in the art can be used to administer these ligands.

There are also disclosed methods for treating and/or preventing estrogen receptor mediated diseases or conditions. By "estrogen receptor mediated diseases or conditions" is meant a physiological or pathological state in which an estrogen receptor is involved. Nonlimiting examples of estrogen receptor mediated diseases or conditions include those of the endocrine system, the reproductive organs, breast tissue, bone tissue, and the vascular system, especially those diseases that become more prevelant in aging males and females. More specifically, such diseases and conditions include those selected from the group consisting of abnormal bone resorption, cardiovascular disease, cancer, metabolic disorders, and central nervous system disorders. Even more specifically, such diseases and conditions include those selected from the group consisting of osteoporosis, breast cancer, uterine cancer, ovarian cancer, prostate cancer, diabetes, and Alzheimer's disease.

### EXAMPLES

The following examples further describe and demonstrate embodiments within the scope of the present invention.

### EXAMPLE 1

### Cloning and Sequencing of cDNA Clones of a Human Estrogen Receptor Gene

The 5' rapid amplification of cDNA ends (RACE) product was identified by performing two rounds of polymerase chain reactions (PCR) on human testis Marathen-Ready cDNA (Clontech product #7414-1) using Vent Polymerase (New England Biolabs product #254S). The first round of PCR was performed using the oligonucleotide, GGAGAAAGCxTGCCCAGGTGTTGGCC (SEQ ID NO: 3), in the 5' coding region of human estrogen receptor beta (GenBank sequence number X99101) and the Clontech AP1 primer, according to the manufacturer's instructions. The second round of PCR was performed using either of two different nested primers having the sequences GTGGTCTGCCGACCAGGCCCACC (SEQ ID NO: 4) or GGTGTTGGCCACAACACATTTGG (SEQ ID NO: 5), corresponding to the 5' end of a human estrogen receptor beta clone (GenBank sequence number X99101), and the Clontech AP2 primer, according to the manufacturer's instructions. The PCR product was subcloned into the PCRAmpScript vector (Stratagene product # 211188) in JM109 E. coli. This clone was sequenced on both strands by cycle sequencing (Pharmacia product #27-1694-01), according to the manufacturer's instructions using primers corresponding to the vector sequence having the following sequence GTAATACGACTCACTATAGGGC (SEQ ID NO: 6) as well as a primer in the 5' end of the human estrogen receptor beta receptor gene having the following sequence GTTAGTGACATTGCTGGGAATGC (SEQ ID NO: 7). Further sequencing was performed with four additional primers having the following sequences: GATCAGAGGCTTCAGCGAAACAG (SEQ ID NO: 8), GAACGCGTGGATTAGTGACTAGCC (SEQ ID NO: 9), GGAGGAAGGAGAATTAAGGCTAG (SEQ ID NO: 10), and GAGATAACAGCTGAGAAAACACC (SEQ ID NO: 11). These four primers were derived from the initial sequence analysis. Sequence alignments and analysis of the nucleotide and protein sequences were carried out using MacVector and AssemblyLign programs (Oxford Molecular Group) as well as the GCG Sequence Analysis Software Package (Madison, WI: pileup).

### EXAMPLE 2

### Cloning and Sequencing of Genomic DNA Clones of a Human Estrogen Receptor Gene

To obtain a probe for use in the screening of a human genomic DNA library, cDNA was first generated from human testis mRNA (Clontech product #6535-1) using an oligo-dT primer and MMLV Reverse Transcriptase (Stratagene product #200420) according to the manufacturer's instructions. The cDNA was amplified by PCR using Boehringer Mannheim's Expand High Fidelity PCR System (product #1 732 641) and two primers having the following sequences:
GTGATGAATTACAGCATTCCCAGCAATGTCACTAACTTGGAAGG (SEQ ID NO: 12) and
ATGGCCCAAGCTTGGGTTCCAGTTCACCTCAGGGCCAGGCG (SEQ ID NO: 13). The PCR product was cloned into the TGEM vector (Promega product #A3600) in JM109 *E. coli*. The product was sequenced on one strand with a Pharmacia cycle sequencing kit (product #27-1694-01) according to the manufacturer's instructions using nine primers having the following sequences: CTTGGAAGGTGGGCCTGGTCGGC (SEQ ID NO: 14), GGAGAAAGGTGCCCAGGTGTTGGCC (SEQ ID NO: 15, which is identical to SEQ ID NO: 3),
CCGTTGCGCCAGCCCTGTTACTGG (SEQ ID NO: 16),
CGCAAGAGCTGCCAGGCCTGCCG (SEQ ID NO: 17),
CCCCGAGCAGCTAGTGCTCACCC (SEQ ID NO: 18),
CTTGGAGAGCTGTTGGATGGAGG (SEQ ID NO: 19),
CTCTGTGTCAAGGCCATGATCC (SEQ ID NO: 20),
CGTCAGGCATGCGAGTAACAAGGG (SEQ ID NO: 21), and
GCAAGTCCTCCATCACGGGGTCCG (SEQ ID NO: 22), corresponding to the published DNA sequence (Mosselman, S. et al., *ERβ: identification and characterization of a novel human estrogen receptor, FEBS Letters,* 392, pp. 49-53 [1996]). Sequence alignments and analysis of the nucleotide and protein sequences were carried out using MacVector and AssemblyLign programs (Oxford Molecular Group) as well as the GCG Sequence Analysis Software Package (Madison, WI: pileup).

The cDNA clone obtained was digested with the restriction enzymes NcoI and KpnI to obtain an approximately 500 base pair fragment corresponding to the 5' end of the human estrogen receptor beta cDNA (GenBank sequence number X99101). This fragment was labeled with P-32 and used to screen a human genomic DNA library (Stratagene product #946206) as per the manufacturer's instructions. One million bacteriophage plaques were screened and seventeen potential hybridizing phages were chosen. These phages were reamplified and screened using a slightly smaller probe (i.e an approximately 300 base pair fragment generated by digesting the human ERbeta clone with NcoI and PstI). Two positive phages were plaque purified and used for the production of DNA. The phages were digested with NotI and BamHI to generate smaller fragments encoding most of the phage DNA and these were subcloned into pBluescript (Stratagene; GenBank #52324). There were two fragments from one phage of approximately 8.5 and 6kb and two fragments from the other phage of approximately 7.7 and 6.3 kb. The genomic subclones of 8.5 and 7.7 kb were sequenced on both strands with a Pharmacia cycle sequencing kit (product #27-1694-01) according to the manufacturer's instructions using primers derived from the 5'RACE product sequencing (EXAMPLE 1). Sequence alignments and analysis of the nucleotide and protein sequences were carried out using MacVector and AssemblyLign programs (Oxford Molecular Group) as well as the GCG Sequence Analysis Software Package (Madison, WI: pileup).

## Claims

1. An isolated estrogen receptor comprising the amino acid sequence of SEQ ID NO:1 (Figure 1).

2. An isolated estrogen receptor according to Claim 1 that is derived from mammalian cells.

3. An isolated estrogen receptor according to Claim 1 that is derived from human cells.

4. An isolated polynucleotide encoding the estrogen receptor having the amino acid sequence of SEQ ID NO: 1.

5. The polynucleotide according to Claim 4 wherein the polynucleotide is DNA.

6. The DNA according to Claim 5 wherein the DNA is a cDNA.

7. An isolated polynucleotide comprising the nucleotide sequence of SEQ ID NO: 2 (Figure 2.)

8. A vector containing the DNA according to Claim 5.

9. A host cell transformed or transfected with a vector according to Claim 8.

10. A method for producing an estrogen receptor comprising: expressing from a host cell according to Claim 9 the estrogen receptor encoded by said DNA.

11. A method for determining whether a ligand not known to be capable of binding to a human estrogen receptor can bind to a human estrogen receptor, said method comprising:
(a) contacting a mammalian cell, comprising an isolated DNA molecule encoding a human estrogen receptor having the amino acid sequence of SEQ ID NO:1, with the ligand under conditions permitting binding of ligands known to bind to an estrogen receptor,
(b) detecting the presence of any of the ligand bound to a human estrogen receptor, and
(c) determining whether the ligand binds to a human estrogen receptor.

## Patentansprüche

1. Isolierter Estrogen-Rezeptor, umfassend die Aminosäuresequenz von SEQ-ID-Nr. 1 (Figur 1).

2. Isolierter Estrogen-Rezeptor nach Anspruch 1, der von Säugerzellen stammt.

3. Isolierter Estrogen-Rezeptor nach Anspruch 1, der von Humanzellen stammt.

4. Isoliertes Polynukleotid, codierend für den Estrogen-Rezeptor mit der Aminosäuresequenz von SEQ-ID-Nr. 1.

5. Polynukleotid nach Anspruch 4, wobei das Polynukleotid DNA ist.

6. DNA nach Anspruch 5, wobei die DNA eine cDNA ist.

7. Isoliertes Polynukleotid, umfassend die Nukleotidsequenz von SEQ-ID-Nr. 2 (Figur 2).

8. Vektor, enthaltend die DNA nach Anspruch 5.

9. Wirtszelle, transformiert oder transfiziert mit einem Vektor nach Anspruch 8.

10. Verfahren zur Herstellung eines Estrogen-Rezeptors, welches umfasst, dass von einer Wirtszelle nach Anspruch 9 der von jener DNA codierte Estrogen-Rezeptor exprimiert wird.

11. Verfahren zur Feststellung, ob ein Ligand, von dem nicht bekannt ist, ob er zur Bindung an einen humanen Estrogen-Rezeptor in der Lage ist, an einen humanen Estrogen-Rezeptor binden kann, welches Verfahren umfasst:
a) Kontaktieren einer Säugerzelle, umfassend ein isoliertes DNA-Molekül, das für einen humanen Estrogen-Rezeptor mit der Aminosäuresequenz von SEQ-ID-Nr. 1 codiert, mit dem Liganden unter Bedingungen, welche die Bindung von Liganden, die bekanntermaßen an einen Estrogen-Rezeptor binden, gestatten,
b) Nachweisen der Anwesenheit von etwaigem Liganden, der an einen humanen Estrogen-Rezeptor gebunden hat, und
c) Feststellen, ob der Ligand an einen humanen Estrogen-Rezeptor bindet.

## Revendications

1. Récepteur oestrogénique isolé comprenant la séquence d'aminoacides de SEQ ID n°: 1 (figure 1).

2. Récepteur oestrogénique isolé selon la revendication 1, qui est dérivé de cellules de mammifères.

3. Récepteur oestrogénique isolé selon la revendication 1, qui est dérivé de cellules humaines.

4. Polynucléotide isolé codant pour le récepteur oestrogénique ayant la séquence d'aminoacides de SEQ ID n°: 1.

5. Polynucléotide selon la revendication 4, le polynucléotide étant un ADN.

6. ADN selon la revendication 5, l'ADN étant un ADNc.

7. Polynucléotide isolé comprenant la séquence nucléotidique de SEQ ID n°: 2 (figure 2).

8. Vecteur contenant l'ADN selon la revendication 5.

9. Cellule hôte transformée ou transfectée avec un vecteur selon la revendication 8.

10. Méthode pour la production d'un récepteur oestrogénique comprenant : l'expression, à partir d'une cellule hôte selon la revendication 9, du récepteur oestrogénique codé par ledit ADN.

11. Méthode pour déterminer si un ligand, dont on ne sait pas s'il est capable de se lier à un récepteur oestrogénique humain, peut se lier à un récepteur oestrogénique, humain, ladite méthode comprenant :
(a) la mise en contact d'une cellule de mammifère, comprenant une molécule d'ADN isolée codant pour un récepteur oestrogénique humain ayant la séquence d'aminoacides de SEQ ID n°: 1, avec le ligand dans des conditions permettant la liaison de ligands dont on sait qu'ils se lient à un récepteur oestrogénique,
(b) la détection de la présence de tout ligand lié à un récepteur oestrogénique humain, et
(c) la détermination du fait que le ligand se lie ou non à un récepteur oestrogénique humain.


### SEQUENCE LISTING

<110> WILKINSON, HILARY
<120> ESTROGEN RECEPTOR
<130> 20047Y
<150> 60/058,271
   <151> 1997-09-08
<150> 60/060,520
   <151> 1997-09-30
<160> 22
<170> FastSEQ for Windows Version 3.0
<210> 1
   <211> 548
   <212> PRT
   <213> HUMAN
<400> 1
   Met Thr Phe Val Ala Ser Ser Cys Lys Val Phe Ser Gln Leu Leu Ser
    1               5                  10                  15
   Gln Asp Met Asp Ile Lys Asn Ser Pro Ser Ser Leu Asn Ser Pro Ser
               20                  25                  30
   Ser Tyr Asn Cys Ser Gln Ser Ile Leu Pro Leu Glu His Gly Ser Ile
           35                  40                  45
   Tyr Ile Pro Ser Ser Tyr Val Asp Ser His His Glu Tyr Pro Ala Met
       50                  55                  60
   Thr Phe Tyr Ser Pro Ala Val Met Asn Tyr Ser Ile Pro Ser Asn Val
   65                  70                  75                  80
   Thr Asn Leu Glu Gly Gly Pro Gly Arg Gln Thr Thr Ser Pro Asn Val
                   85                  90                  95
   Leu Trp Pro Thr Pro Gly His Leu Ser Pro Leu Val Val His Arg Gln
               100                 105                 110
   Leu Ser His Leu Tyr Ala Glu Pro Gln Lys Ser Pro Trp Cys Glu Ala
           115                 120                 125
   Arg Ser Leu Glu His Thr Leu Pro Val Asn Arg Glu Thr Leu Lys Arg
       130                 135                 140
   Lys Val Ser Gly Asn Arg Cys Ala Ser Pro Val Thr Gly Pro Gly Ser
   145                 150                 155                 160
   Lys Arg Asp Ala His Phe Cys Ala Val Cys Ser Asp Tyr Ala Ser Gly
                   165                 170                 175
   Tyr His Tyr Gly Val Trp Ser Cys Glu Gly Cys Lys Ala Phe Phe Lys
               180                 185                 190
   Arg Ser Ile Gln Gly His Asn Asp Tyr Ile Cys Pro Ala Thr Asn Gln
           195                 200                 205
   Cys Thr Ile Asp Lys Asn Arg Arg Lys Ser Cys Gln Ala Cys Arg Leu
       210                 215                 220
   Arg Lys Cys Tyr Glu Val Gly Met Val Lys Cys Gly Ser Arg Arg Glu
   225                 230                 235                 240
   Arg Cys Gly Tyr Arg Leu Val Arg Arg Gln Arg Ser Ala Asp Glu Gln
                   245                 250                 255
   Leu His Cys Ala Gly Lys Ala Lys Arg Ser Gly Gly His Ala Pro Arg
               260                 265                 270
   Val Arg Glu Leu Leu Leu Asp Ala Leu Ser Pro Glu Gln Leu Val Leu
           275                 280                 285
   Thr Leu Leu Glu Ala Glu Pro Pro His Val Leu Ile Ser Arg Pro Ser
       290                 295                 300
   Ala Pro Phe Thr Glu Ala Ser Met Met Met Ser Leu Thr Lys Leu Ala
   305                 310                 315                 320
   Asp Lys Glu Leu Val His Met Ile Ser Trp Ala Lys Lys Ile Pro Gly
                   325                 330                 335
   Phe Val Glu Leu Ser Leu Phe Asp Gln Val Arg Leu Leu Glu Ser Cys
               340                 345                 350
   Trp Met Glu Val Leu Met Met Gly Leu Met Trp Arg Ser Ile Asp His
           355                 360                 365
   Pro Gly Lys Leu Ile Phe Ala Pro Asp Leu Val Leu Asp Arg Asp Glu
       370                 375                 380
   Gly Lys Cys Val Glu Gly Ile Leu Glu Ile Phe Asp Met Leu Leu Ala
   385                 390                 395                 400
   Thr Thr Ser Arg Phe Arg Glu Leu Lys Leu Gln His Lys Glu Tyr Leu
                   405                 410                 415
   Cys Val Lys Ala Met Ile Leu Leu Asn Ser Ser Met Tyr Pro Leu Val
               420                 425                 430
   Thr Ala Thr Gln Asp Ala Asp Ser Ser Arg Lys Leu Ala His Leu Leu
           435                 440                 445
   Asn Ala Val Thr Asp Ala Leu Val Trp Val Ile Ala Lys Ser Gly Ile
       450                 455                 460
   Ser Ser Gln Gln Gln Ser Met Arg Leu Ala Asn Leu Leu Met Leu Leu
   465                 470                 475                 480
   Ser His Val Arg His Ala Ser Asn Lys Gly Met Glu His Leu Leu Asn
                   485                 490                 495
   Met Lys Cys Lys Asn Val Val Pro Val Tyr Asp Leu Leu Leu Glu Met
               500                 505                 510
   Leu Asn Ala His Val Leu Arg Gly Cys Lys Ser Ser Ile Thr Gly Ser
           515                 520                 525
   Glu Cys Ser Pro Ala Glu Asp Ser Lys Ser Lys Glu Gly Ser Gln Asn
       530                 535                 540
   Pro Gln Ser Gln
   545
<210> 2
   <211> 1647
   <212> DNA
   <213> HUMAN
<400> 2
   atgacctttg tagcctcttc ttgcaaggtg ttttctcagc tgttatctca agacatggat     60
   ataaaaaact caccatctag ccttaattct ccttcctcct acaactgcag tcaatccatc    120
   ttacccctgg agcacggctc catatacata ccttcctcct atgtagacag ccaccatgaa    180
   tatccagcca tgacattcta tagccctgct gtgatgaatt acagcattcc cagcaatgtc    240
   actaacttgg aaggtgggcc tggtcggcag accacaagcc caaatgtgtt gtggccaaca    300
   cctgggcacc tttctccttt agtggtccat cgccagttat cacatctgta tgcggaacct    360
   caaaagagtc cctggtgtga agcaagatcg ctagaacaca ccttacctgt aaacagagag    420
   acactgaaaa ggaaggttag tgggaaccgt tgcgccagcc ctgttactgg tccaggttca    480
   aagagggatg ctcacttctg cgctgtctgc agcgattacg catcgggata tcactatgga    540
   gtctggtcgt gtgaaggatg taaggccttt tttaaaagaa gcattcaagg acataatgat     600
   tatatttgtc cagctacaaa tcagtgtaca atcgataaaa accggcgcaa gagctgccag     660
   gcctgccgac ttcggaagtg ttacgaagtg ggaatggtga agtgtggctc ccggagagag     720
   agatgtgggt accgccttgt gcggagacag agaagtgccg acgagcagct gcactgtgcc     780
   ggcaaggcca agagaagtgg cggccacgcg ccccgagtgc gggagctgct gctggacgcc     840
   ctgagccccg agcagctagt gctcaccctc ctggaggctg agccgcccca tgtgctgatc     900
   agccgcccca gtgcgccctt caccgaggcc tccatgatga tgtccctgac caagttggcc     960
   gacaaggagt tggtacacat gatcagctgg gccaagaaga ttcccggctt tgtggagctc    1020
   agcctgttcg accaagtgcg gctcttggag agctgttgga tggaggtgtt aatgatgggg    1080
   ctgatgtggc gctcaattga ccaccccggc aagctcatct ttgctccaga tcttgttctg    1140
   gacagggatg aggggaaatg cgtagaagga attctggaaa tctttgacat gctcctggca    1200
   actacttcaa ggtttcgaga gttaaaactc caacacaaag aatatctctg tgtcaaggcc    1260
   atgatcctgc tcaattccag tatgtaccct ctggtcacag cgacccagga tgctgacagc    1320
   agccggaagc tggctcactt gctgaacgcc gtgaccgatg ctttggtttg ggtgattgcc    1380
   aagagcggca tctcctccca gcagcaatcc atgcgcctgg ctaacctcct gatgctcctg    1440
   tcccacgtca ggcatgcgag taacaagggc atggaacatc tgctcaacat gaagtgcaaa    1500
   aatgtggtcc cagtgtatga cctgctgctg gagatgctga atgcccacgt gcttcgcggg    1560
   tgcaagtcct ccatcacggg gtccgagtgc agcccggcag aggacagtaa aagcaaagag    1620
   ggctcccaga acccacagtc tcagtga                                        1647
<210> 3
   <211> 25
   <212> DNA
   <213> HUMAN
<400> 3
   ggagaaaggt gcccaggtgt tggcc 25
<210> 4
   <211> 23
   <212> DNA
   <213> HUMAN
<400> 4
   gtggtctgcc gaccaggccc acc 23
<210> 5
   <211> 23
   <212> DNA
   <213> HUMAN
<400> 5
   ggtgttggcc acaacacatt tgg 23
<210> 6
   <211> 22
   <212> DNA
   <213> HUMAN
<400> 6
   gtaatacgac tcactatagg gc 22
<210> 7
   <211> 23
   <212> DNA
   <213> HUMAN
<400> 7
   gttagtgaca ttgctgggaa tgc 23
<210> 8
   <211> 23
   <212> DNA
   <213> HUMAN
<400> 8
   gatcagaggc ttcagcgaaa cag 23
<210> 9
   <211> 24
   <212> DNA
   <213> HUMAN
<400> 9
   gaacgcgtgg attagtgact agcc 24
<210> 10
   <211> 23
   <212> DNA
   <213> HUMAN
<400> 10
   ggaggaagga gaattaaggc tag 23
<210> 11
   <211> 23
   <212> DNA
   <213> HUMAN
<400> 11
   gagataacag ctgagaaaac acc 23
<210> 12
   <211> 44
   <212> DNA
   <213> HUMAN
<400> 12
   gtgatgaatt acagcattcc cagcaatgtc actaacttgg aagg 44
<210> 13
   <211> 41
   <212>. DNA
   <213> HUMAN
<400> 13
   atggcccaag cttgggttcc agttcacctc agggccaggc g 41
<210> 14
   <211> 23
   <212> DNA
   <213> HUMAN
<400> 14
   cttggaaggt gggcctggtc ggc 23
<210> 15
   <211> 25
   <212> DNA
   <213> HUMAN
<400> 15
   ggagaaaggt gcccaggtgt tggcc 25
<210> 16
   <211> 24
   <212> DNA
   <213> HUMAN
<400> 16
   ccgttgcgcc agccctgtta ctgg 24
<210> 17
   <211> 23
   <212> DNA
   <213> HUMAN
<400> 17
   cgcaagagct gccaggcctg ccg 23
<210> 18
   <211> 23
   <212> DNA
   <213> HUMAN
<400> 18
   ccccgagcag ctagtgctca ccc 23
<210> 19
   <211> 23
   <212> DNA
   <213> HUMAN
<400> 19
   cttggagagc tgttggatgg agg 23
<210> 20
   <211> 22
   <212> DNA
   <213> HUMAN
<400> 20
   ctctgtgtca aggccatgat cc 22
<210> 21
   <211> 24
   <212> DNA
   <213> HUMAN
<400> 21
   cgtcaggcat gcgagtaaca aggg 24
<210> 22
   <211> 24
   <212> DNA
   <213> HUMAN
<400> 22
   gcaagtcctc catcacgggg tccg 24